# EUROPEAN PATENT APPLICATION

(11) **EP 4 545 102 A1**
(43) Date of publication of application: **30.04.2025**
(21) Application number: 23383088.4
(22) Date of filing: 24.10.2023
(51) Int. Cl.: A61L 9/22, B03C 3/38, B03C 3/41, F24F 8/30, H01T 23/00, A01N 25/00, A01P 1/00, A61B 90/80, C05D 1/00, H05H 1/00, A61L 2/00, A61L 2/18

(54) **SYSTEM AND METHOD FOR STERILIZING AN AIR VOLUME**

(71) Applicant: Instituto Nacional De Tecnica Aeroespacial "Esteban Terradas", 28850 Torrejón de Ardoz (ES)
(72) Inventor: CONESA TORRES, Antonio Jesús, 28850 Torrejón de Ardoz (ES); CABRERA REVUELTA, Ángel, 28850 Torrejón de Ardoz (ES); SÁNCHEZ GARCÍA, Mario, 28850 Torrejón de Ardoz (ES); GONZÁLEZ DE FIGUERAS, Carolina, 28850 Torrejón de Ardoz (ES); GONZÁLEZ PASTOR, José Eduardo, 28850 Torrejón de Ardoz (ES)
(74) Representative: TRBL Intellectual Property

(57) **Abstract**

The invention presents a device for sterilizing. The device comprises a first element (1) suitable for receiving a working substance and a first group of external electrodes (2). Each first external electrode comprises a first discharge zone (3, 30) which is arranged at a first discharge distance from the first element, the first discharge zone being the zone of the first external electrode being closest to the first element. The device further comprises an internal electrode (6) configured to be arranged in the first element (1) and a power unit (7) connected to the first group of external electrodes, wherein the power unit (7) is configured to establish a first voltage between the first group of external electrodes and the internal electrode. Finally, the device comprises an airflow generator (8) configured to create an airflow in a region comprised between the first discharge zone and the first element (1). The invention also provides a method for sterilizing a volume using such a device.

## Description

### TECHNICAL FIELD

This invention belongs to the field of devices and methods intended to sterilize an air volume and/or surface, the method being also valid for insect plagues eradication.

### STATE OF THE ART

There are a vast number of devices which are intended to sterilize air volumes, killing bacteria or any other microorganisms which may be harmful to human beings.

Some of them activate water to pour it into surfaces which are to be sterilized. These devices are effective, but only to sterilize surfaces. Documents such as EP 3470364 A1 disclose this type of devices, where plasma nozzles are used to create activated water, which is used to disinfect a selected surface.

Other type of devices are reactor-type devices which keep a sterilized inner volume, but require very demanding features, such as pressure and temperature. For example, document DE 10 2008 037 898 A1 discloses a method and a device for disinfecting or sterilizing some objects, which are treated with a gas generated in a plasma reactor.

The present invention provides an advantageous device which overcome the aforementioned drawbacks.

### DESCRIPTION OF THE INVENTION

The invention provides an alternative solution for sterilizing an air volume by means of a system according to claim 1. Preferred embodiments of the invention are defined in dependent claims.

Unless otherwise defined, all terms (including technical and scientific terms) used herein are to be interpreted as is customary in the art. It will be further understood that terms in common usage should also be interpreted as is customary in the relevant art and not in an idealised or overly formal sense unless expressly so defined herein.

In this text, the term "comprises" and its derivations (such as "comprising", etc.) should not be understood in an excluding sense, that is, these terms should not be interpreted as excluding the possibility that what is described and defined may include further elements, steps, etc.

In a first inventive aspect, the invention provides a device for sterilizing comprising
a first element suitable for receiving a working substance;
a first group of external electrodes, comprising at least one first external electrode, each first external electrode comprising a first discharge zone which is arranged at a first discharge distance from the first element, the first discharge zone being the zone of the first external electrode being closest to the first element;
an internal electrode configured to be arranged in the first element;
a power unit connected to the first group of external electrodes, wherein the power unit is configured to establish a first voltage between the first group of external electrodes and the internal electrode;
an airflow generator configured to create an airflow in a region comprised between the first discharge zone and the first element.

The first element is a generic element that is used to receive a working substance. In some cases, the working substance will be a liquid, so the first element will be a receptacle to receive the liquid. However, in other cases the working substance may be a more viscous fluid, or gel mixture. In these cases, the first element may be a cartridge or just a plane sheet. In any of the mentioned examples, it is clear that the first element provides a support and a position for the working substance that is going to interact with the electrodes.

The first group of external electrodes comprises at least one electrode. Hence, each electrode comprises a discharge zone, which is the zone which is closest to the first element. The internal electrode is intended to be submerged or introduced in the working substance, so its position is closest to the first element than in the case of the first external electrodes, which are not intended to be in contact with the working substance. The working substance is not included in the definition of this inventive aspect, since the device is described "on the shelf".

This device is configured to create plasma region between the electrodes and the working substance, thus creating a sterilization atmosphere wherein microorganisms or other living creatures, such as plague insects, are killed. Further, the plasma actuation creates an activated working substance which can be used, e.g., to enhance the development and growing of plants and vegetables.

In some particular embodiments, the device further comprises a second group of external electrodes, comprising at least one second external electrode, each second external electrode comprising a second discharge zone which is arranged at a second discharge distance from the first element, the second discharge zone being the zone of the second external electrode being closest to the first element. In these embodiments, the power unit is further connected to the second group of external electrodes and is further configured to establish a second voltage between the second group of external electrodes and the internal electrode. In these embodiments, the airflow generator is further configured to create an airflow in a region comprised between the second discharge zone and the first element.

A second group of electrodes contributes to a bigger plasma region, thus increasing the efficiency of the device.

In some particular embodiments, the first voltage and the second voltage have different polarities.

The different polarity of the first and second voltages is aimed to obtain plasma with different reactive elements, so that they complement each other. The power unit may be a single complex unit with two outputs (one for each group of electrodes) or an arrangement of two different power sources (one for each group of electrodes).

In some particular embodiments, the first group of external electrodes and/or the second group of external electrodes comprises a plurality of electrodes.

A plurality of electrodes is not strictly necessary, but improves the efficiency of the device, ensuring that the discharge is applied to the working substance in the first element.

In some particular embodiments, the device comprises a further airflow generator, so that one airflow generator is oriented to one discharge zone and the other airflow generator is oriented to the other discharge zone.

In some particular embodiments, the airflow generator is a fan which comprises a speed control.

The fan accelerates the dispersion of the plasma, thus improving the efficiency of the device. The speed of the fan causes an effect in the plasma.

In some particular embodiments, the device further comprises a plurality of walls creating an inner space, wherein the first element and the electrodes are comprised in the inner space.

The inner space creates a more controlled environment, increasing the concentration of plasma reactive species and thus improving the efficiency of the device.

In some particular embodiments, the walls are assembled with a watertight closure.

The watertight closure, which is not essential, is advantageous when a rapid actuation is needed.

In some particular embodiments, the discharge zone is a tip or an end of a rod.

Different configurations may be defined for the electrodes to achieve an advantageous discharge zone. In some cases, a thin rod is arranged, so that the tip is the closest zone of the rod to the first element. In other cases, a thin rod may be arranged, but in such a way that the closest zone is not the tip, but an intermediate bended portion.

In some particular embodiments, the external electrodes comprise at least one of carbon, copper, aluminium, tin and/or stainless steel. However, other conductive materials are also suitable for this purpose.

In a further inventive aspect, the invention provides a method to sterilize an air volume, the method comprising the steps of
providing a device according to the first inventive aspect, wherein the first element and the electrodes are arranged in the air volume to be sterilized;
provide the first element with a working substance comprising water, wherein the working substance defines a working surface, which is the surface of the working substance which is closest to the electrodes;
arrange the first group of external electrodes and the second group of external electrodes so that the first and second discharge zones are located at a distance lower than 30 mm with respect to the working surface;
arrange the internal electrode in contact with the working substance;
apply a first voltage lower than +100 kV;
apply a second voltage lower than -100 kV; and
activate the airflow generator to direct an airflow towards a region comprised between at least one of the discharge zones and the working surface.

A Voltage lower than -100 kV should be understood as a voltage with an absolute value lower than 100 kV and with an inverse polarity with respect to the first voltage.

This method creates a plasma discharge above the working substance comprised in the first element. This working substance, as explained above, can be a liquid (so the first element will be a receptacle for this liquid) or can be a more viscous fluid, such as a gel (so the first element does not need to be a proper receptacle and can be a cartridge or just a plate or sheet). In any case, the working substance defines a working surface. In the case of the liquid, the surface of the liquid will be the working surface and in the event of a more viscous fluid, the surface of the working substance which is faced to the electrodes, closest to them, will be the working surface, which is intended to interact with the electrodes. The products and reactive species generated by this plasma extend to the air volume to be sterilized, killing bacteria or other microorganisms, as well as some insect plagues. The airflow generator accelerates the dispersion of the plasma, thus improving the efficiency of the device. The region between the discharge zone and the working surface contains at least one line defined by the shortest path from the discharge zone to the liquid surface, so the skilled person would perfectly understand which is the zone between the first (or second) discharge zone and the receptacle. Depending on the size and power of the airflow generator, and the distance between the first and the second discharge zone, it is possible to reach both zones with the same airflow or change the direction of the airflow to reach alternatively one discharge zone and then the other one.

Further, this method activates the working substance contained in the receptacle, and this activated working substance can be used later on to improve the development of some vegetables. Hence, in some particular embodiments, the working substance is a liquid and the method comprises the step of using the activated substance to water a vegetable species. In other particular embodiments, the working substance is a liquid and the method comprises the step of using the activated substance to disinfect a surface, such as animal or human skin.

In some particular embodiments, the first voltage and/or the second voltage is direct or pulsed. Although alternate current may also be used, Using direct current is less likely to cause electromagnetic interference to other electronic devices.

In some particular embodiments, the working substance comprises phosphorus or potassium. These elements, and the compounds that comprise them, can be advantageous in some fertilizing applications when combined with the nitrogen species obtained in the operation of the device.

In some particular embodiments, the device further comprises a plurality of walls creating an inner space and the air volume to be sterilized is arranged in the inner space.

In some particular embodiments,
the first voltage, in KV, is equal or higher than 30 times the distance from the first discharge zones and the working surface, in cm; and
the second voltage, in KV, is equal or higher than 30 times the distance from the second discharge zones and the working surface, in cm.

The dielectric strength of air is, approximately, 30kV per cm, so the voltage should be enough to cause break down.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a schematic view of a device according to the invention.
Figure 2a and 2b show some alternatives in the discharge zones of the electrodes.
Figure 3 shows an example of the use of a device according to the invention in a greenhouse.
Figure 4 shows an example of the use of a device according to the invention in an operation theatre.

### DETAILED DESCRIPTION OF THE INVENTION

The example embodiments are described in sufficient detail to enable those of ordinary skill in the art to embody and implement the systems and processes herein described. It is important to understand that embodiments can be provided in many alternate forms and should not be construed as limited to the examples set forth herein.

Accordingly, while embodiment can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

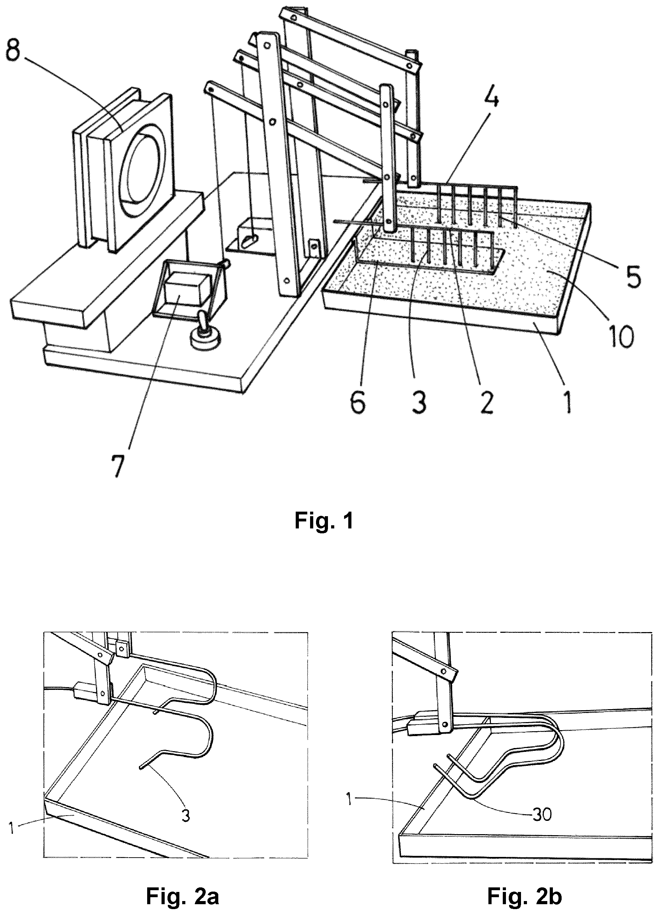

Figure 1 shows a schematic view of a device according to the invention.

This device comprises a first element 1 suitable for receiving a working substance. In this case, the first element is a receptacle 1 and the working substance is an aqueous solution 10, which defines a water surface.

The device further comprises a first group of external electrodes, comprising five first external electrodes 2. Each first external electrode comprises a first discharge zone which is arranged at a tip distance from the water surface. This first discharge distance is 6 mm from the water surface. In this example, the discharge zone of the electrode is the tip 3 of a rod, which is arranged so that it is the closest point of the rod to the water surface.

The device further comprises a second group of external electrodes, comprising five second external electrodes 4. Each second external electrode comprises a second discharge zone which is arranged at a second discharge distance from the water surface. This second discharge distance is 6 mm from the water surface. In this example, the discharge zone of the electrode is the tip 5 of a rod, which is arranged so that it is the closest point of the rod to the water surface.

The device further comprises an internal electrode 6 configured to be submerged in the aqueous solution of the receptacle 1.

The device further comprises a power unit 7 connected to the first group of external electrodes and to the second group of external electrodes. The power unit 7 comprises two power sources, one for each group of electrodes, and is configured to establish a first voltage between the first group of external electrodes and the internal electrode and is configured to establish a second voltage between the second group of external electrodes and the internal electrode.

The device further comprises a fan 8 configured to create an airflow in a region comprised between the tip of the electrodes and the water surface.

Figures 2a and 2b show some alternatives in the discharge zones of the electrodes. Figure 2a shows a first alternative, which is similar to the one used in the device of Figure 1. In this case, the discharge zone is the tip 3 of a rod, which in this case is bended, but can be straight or bended. The tip 3 is the end of the rod and is also the point which is closest to the water surface contained in the receptacle 1. During the operation, once that the electrodes are fed, the discharge is initiated in the discharge zone 3. Due to the airflow, it is possible that the discharge is displaced and stabilized in a zone which is different to the original discharge zone but, once that the discharge is extinguished, it is initiated again in the discharge zone 3.

Figure 2b shows a second alternative. In this case, the electrode comprises a rod, but the discharge zone is not located in the end of the rod, but in a specific zone 30 which is before the tip. This discharge zone 30 is the zone which is closest to the water surface contained in the receptacle 1, but is not the tip or the end of the rod. During the operation, once that the electrodes are fed, the discharge is initiated in the discharge zone 30. Due to the airflow, it is possible that the discharge is displaced and stabilized in a zone which is different to the original discharge zone but, once that the discharge is extinguished, it is initiated again in the discharge zone 30.

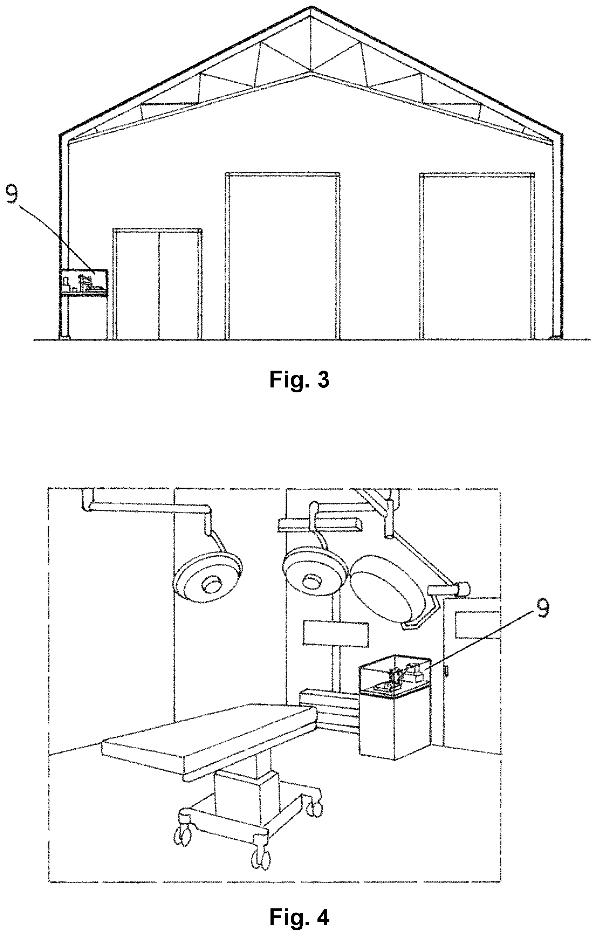

Figure 3 shows a first example of the operation of such a device. In this case, the device is located in a greenhouse, which contains an air volume to be sterilized or disinfected. The walls 9 define this air volume to be sterilized, which contains the elements of the device: a receptacle with water, the first group of electrodes, the second group of electrodes, the internal electrode, the power unit and the fan.

The water defines a water surface, and the electrodes are arranged at a distance of 6 mm with respect to the water surface.

The air volume to be sterilized is then defined between the walls 9, which in this case is not watertight, but in other examples, can be. The power unit applies a first voltage of +20 kV between the first group of external electrodes and the internal electrode. Also, a second voltage of -20 kV is applied by the other power source of the power unit between the second group of external electrodes and the internal electrode.

The relation between the first and second voltages and the first and second discharge distances is defined by the dielectric strength of the air, which is, approximately. 30 kV per cm.

Some plasma is created in the region above the water contained in the receptacle, due to the action of the high voltage between the electrodes and the water. The fan is directed towards a region comprised between the tips of the electrodes and the liquid surface, so that the plasma is directed towards the inner space, thus killing microorganisms (including their resistance forms, bacterial spores)in the inner zone of the greenhouse.

The water that is contained in the receptacle is activated by the action of the electrodes, and this water is at a later stage, used to water the vegetables contained in the greenhouse. This synergic application achieves, on one side, the disinfection of the interior of the greenhouse, thus avoiding insect plagues and other problems related to microorganisms. Further, the vegetables of the greenhouse are watered with activated water, which is obtained in the same act of disinfection, thus improving the growth of the species.

Figure 4 shows an operating theatre where the invention is applied. The walls 9 define the inner space, which contains the elements of the device: a receptacle with a culture medium, the first group of electrodes, the second group of electrodes, the internal electrode, the power unit and the fan.

When the power unit feeds the electrodes with the corresponding voltage, as explained above, plasma is created in the region above the medium surface, and the airflow produced by the fan directs the plasma towards the inner space, thus killing the Microorganisms and bacterial spores in the inner zone of the operation theatre.

The water that is contained in the receptacle is activated by the action of the electrodes, and this water is at a later stage, used to disinfect the skin of patients and doctors. This synergic application achieves, on one side, the disinfection of the interior of the operation theatre, thus avoiding problems related to microorganisms and their spores, which are especially important in this location. Further, a further disinfection effect is achieved on the skin of the people involved in the operation, thus improving the whole outcome of the process.

## Claims

1. Device for sterilizing comprising
a first element (1) suitable for receiving a working substance;
a first group of external electrodes, comprising at least one first external electrode (2), each first external electrode comprising a first discharge zone (3, 30) which is arranged at a first discharge distance from the first element, the first discharge zone being the zone of the first external electrode being closest to the first element;
an internal electrode (6) configured to be arranged in the first element (1);
a power unit (7) connected to the first group of external electrodes, wherein the power unit (7) is configured to establish a first voltage between the first group of external electrodes and the internal electrode; and
an airflow generator (8) configured to create an airflow in a region comprised between the first discharge zone and the first element (1).

2. Device according to claim 1, further comprising a second group of external electrodes, comprising at least one second external electrode (4), each second external electrode comprising a second discharge zone (5) which is arranged at a second discharge distance from the first element (1), the second discharge zone being the zone of the second external electrode being closest to the first element (1); wherein the power unit (7) is also connected to the second group of external electrodes and is configured to establish a second voltage between the second group of external electrodes and the internal electrode; and wherein the airflow generator is further configured to create an airflow in a region comprised between the second discharge zone and the first element (1).

3. Device according to any of the preceding claims, wherein the first voltage and the second voltage have different polarities.

4. Device according to any of the preceding claims, wherein the airflow generator is a fan (8) which comprises a speed control.

5. Device according to any of the preceding claims, further comprising a plurality of walls (9) creating an inner space, wherein the first element and the electrodes are comprised in the inner space.

6. Device according to any of the preceding claims, wherein the discharge zone is a tip or an end of a rod.

7. Device according to any of the preceding claims, wherein the external electrodes comprise at least one of carbon, copper, aluminium, tin and/or stainless steel.

8. Device according to any of the preceding claims, further comprising a further airflow generator, so that one airflow generator is oriented to one discharge zone and the other airflow generator is oriented to the other discharge zone

9. Method to sterilize an air volume, the method comprising the steps of
providing a device according to any of the preceding claims, wherein the first element and the electrodes are arranged in the air volume to be sterilized;
provide the first element with a working substance comprising water, wherein the working substance defines a working surface, which is the surface of the working substance which is closest to the electrodes;
arrange the first group of external electrodes and the second group of external electrodes so that the first and second discharge zones are located at a distance lower than 30 mm with respect to the working surface;
arrange the internal electrode (6) in contact with the working substance;
apply a first voltage lower than +100 kV;
apply a second voltage lower than -100 kV; and
activate the airflow generator to direct an airflow towards a region comprised between at least one of the discharge zones and the working surface.

10. Method according to claim 9, wherein the working substance comprises phosphorus or potassium.

11. Method according to any of claims 9 or 10, wherein the working substance is a liquid or a gel.

12. Method according to any of claims 9 to 11, wherein the working substance is a liquid and the method comprises the step of using the activated substance to water a vegetable species.

13. Method according to any of claims 9 to 11, wherein the working substance is a liquid and the method comprises the step of using the activated substance to disinfect a surface, such as animal or human skin.

14. Method according to any of claims 9 to 13, wherein the device is a device according to claim 5 and the air volume to be sterilized is arranged in the inner space.

15. Method according to any of claims 9 to 14, wherein
the first voltage, in KV, is equal or higher than 30 times the distance from the first discharge zones and the working surface, in cm; and
the second voltage, in KV, is equal or higher than 30 times the distance from the second discharge zones and the working surface, in cm.
